Europäisches Patentamt

European Patent Office

Office européen des brevets

(11) Veröffentlichungsnummer : **0 468 247 B1**

(12) **EUROPÄISCHE PATENTSCHRIFT**

(45) Veröffentlichungstag der Patentschrift :
**01.06.94 Patentblatt 94/22**

(51) Int. Cl.$^5$ : **A61K 9/50,** A61K 9/20,
A61K 31/455

(21) Anmeldenummer : **91111146.6**

(22) Anmeldetag : **04.07.91**

(54) Verfahren zur Herstellung mechanisch stabiler, gut zerfallender Komprimate aus kleindimensionierten Extrusionspellets mit hohem Etofibratgehalt.

(30) Priorität : **07.07.90 DE 4021678**

(43) Veröffentlichungstag der Anmeldung :
**29.01.92 Patentblatt 92/05**

(45) Bekanntmachung des Hinweises auf die
Patenterteilung :
**01.06.94 Patentblatt 94/22**

(84) Benannte Vertragsstaaten :
**DE ES FR GB IT**

(56) Entgegenhaltungen :
**EP-A- 218 928**
**EP-A- 226 884**
**Derwent file supplier WPIL, 1987 AN 87-099083**
**(14), Derwent Publications Ltd, London, GB**

(73) Patentinhaber : **Merz & Co. GmbH & Co.**
**Eckenheimer Landstrasse 100-104**
**D-60318 Frankfurt (DE)**

(72) Erfinder : **Nürnberg, Eberhard, Prof.Dr.**
**Ruhsteinweg 18**
**W-8525 Uttenreuth/Weiher (DE)**
Erfinder : **Seiller, Erhard, Dr.**
**Am Weinberg 15a**
**W-6368 Bad Vilbel 3 (DE)**
Erfinder : **Kühn, Bernd**
**Am Europakanal 14**
**W-8520 Erlangen/Büchenbach (DE)**

(74) Vertreter : **Beil, Hans Chr., Dr. et al**
**BEIL, WOLFF & BEIL,**
**Rechtsanwälte,**
**Postfach 80 01 40**
**D-65901 Frankfurt (DE)**

## Beschreibung

Die Erfindung betrifft ein Verfahren zur Herstellung von Komprimaten gemäß dem Oberbegriff des Patentanspruchs 1.

In der DE-A 40 21 678.0 wurde ein Verfahren zur Herstellung kleindimensionierter, oral applizierbarer Formkörper mit hohem Gehalt an Etofibrat und guter Verträglichkeit sowie kontinuierlicher Wirkstoff-Freisetzung beschrieben. Kennzeichnend für dieses Verfahren ist die Herstellung eines Extrudats aus einem Gemisch von zwei Kolloiden mit unterschiedlicher Wasserlöslichkeit und ungefähr 80 % Etofibrat. Die Extrusionsformkörper werden in kleine zylindrische Teile geschnitten und gegebenenfalls ausgerundet, um dann entweder in Hartkapseln abgefüllt oder zu Tabletten gepreßt zu werden.

Bei der Tablettierung von ausgerundeten oder zylindrischen Extrusionspellets mit einem hohem Gehalt an Etofibrat wurde beobachtet, daß man entweder bei Anwendung hoher Preßkräfte Tabletten mit einem verzögerten Zerfall erhält oder bei Anwendung niedriger Preßkräfte Komprimate, die zwar nach kurzer Zeit in kleine Formkörper zerfallen, aber vorher (in trockener Form) eine nicht befriedigende mechanische Widerstandsfähigkeit aufweisen. Im erstgenannten Fall würde die Wirkstoff-Freisetzung und damit die pharmakokinetischen Eigenschaften der Arzneiform insofern verändert, als eine Inhibierung der Freisetzung zu veränderten Blutspiegelwerten führen muß. Im zweitgenannten Fall, bei dem gut zerfallende Tabletten erhalten werden, erhält man Komprimate, die bei der Verarbeitung, wie beispielsweise bei der Abfüllung in Blisterpackungen oder andere Behältnisse bereits Beschädigungen erfahren können. Eine derartige Zubereitung ist für den praktischen Gebrauch daher nicht optimal.

Gegenstand der Erfindung ist ein Verfahren zur Herstellung hochdosierter Etofibratkomprimate aus Extrusionspellets mit einem raschen Zerfall in Formkörper, die dem Ausgangsmaterial, also den Pellets, entsprechen. Bei der Lösung dieser Aufgabe sollte eine Tablette gewonnen werden, die eine hinreichende Abriebfestigkeit (Friabilität) aufweist und die Freisetzungseigenschaften des Wirkstoffs aus den Pellets nur unwesentlich verändert. Es wurde überraschend gefunden, daß diese Aufgabe dadurch gelöst werden kann, daß die kleindimensionierten Extrusionsformkörper mit einem Überzug aus folgenden Komponenten versehen werden:

- wasserlöslicher Polyvinylalkohol mit einem Estergehalt (wie beispielsweise Polyvinylacetat) von 19,4 bis 6,7 %
- Sprengmittel
- Cellulose
- gegebenenfalls weiteren Kolloiden, Hilfs-, Farb- und/oder Aromastoffen.

Diese Komponenten werden in Form einer wäßrigen oder wäßrig-organischen Suspension, die 0,1 bis 20, vorzugsweise 4-7 Gew.-% Polyvinylalkohol, 0,1-20, vorzugsweise 4-7 Gew.-% Sprengmittel, 0,1-20, vorzugsweise 4-7 Gew.-% Cellulose, 0-20 Gew.-% Kolloide, 0-20 Gew.-% Hilfsstoffe, 0-5 Gew.-% Farbstoffe und/oder 0-20 Gew.-% Aromastoffe enthält, auf die Extrusionspellets nach bekannten Verfahrensweisen aufgetragen. Der Auftrag kann beispielsweise in mehreren Schritten durch Aufsprühen in einem Dragierkessel mit zwischengeschalteten Trocknungsschritten oder auch durch kontinuierliches Besprühen und anschließendes Trocknen erfolgen. Im Hinblick auf die Temperaturempfindlichkeit des Wirkstoffs Etofibrat darf die Temperatur der Zuluft bei der Trocknung 40°C nicht überschreiten. Die Überzugsschicht soll so stark sein, daß sie 1-60 Gew.-%, bezogen auf die überzogenen Pellets, beträgt.

Die überzogenen Extrusionspellets können dann nach bekannten Verfahren, beispielsweise auf einer Exzenter- oder Rundläuferpresse, zu Komprimaten mit dem gewünschten Wirkstoffgehalt verpreßt werden. Die anzuwendenden Preßkräfte liegen zwischen 2 und 10 kN, vorzugsweise 3 bis 5 kN. Die erhaltenen Komprimate haben eine ausreichende Bruch- und Abriebfestigkeit und zerfallen in Wasser sehr schnell, nämlich in 5 bis 300 s, vorzugsweise 15 bis 60 s, wieder in die ursprünglichen Extrusionspellets. Die pharmakokinetischen Eigenschaften entsprechen damit denjenigen der verwendeten Extrusionspellets und denjenigen von Hartkapseln, die mit den gleichen Etofibrat-Extrusionspellets gefüllt sind.

Charakteristisch für das erfindungsgemäße Verfahren ist, daß die auf die Extrusionspellets aufgetragenen Überzüge nicht eine völlig glatte, sondern elektronenmikroskopisch gut erkennbar rauhe Oberfläche besitzen. Dadurch wird eine gute Verhakung der einzelnen Teilchen nach dem Verpressen zu Komprimaten mit niedrigen Preßkräften bewirkt.

Die erhaltenen Komprimate können, falls dies gewünscht wird, ihrerseits mit einem Überzug versehen werden. Dieser kann aus einer filmbildenden Substanz, gegebenenfalls unter Zusatz von Weichmachern, Gleitmitteln sowie weiteren Hilfsstoffen, wie Pigmenten, Zucker oder anderen Süßstoffen, Farb- und/oder Aromastoffen bestehen.

Als erfindungsgemäß einzusetzendes Sprengmittel kommt insbesondere quervernetztes Polyvinylpyrrolidon in Betracht. Die Cellulose wird vorzugsweise in Form von Cellulosepulver und/oder mikrokristalliner Cellulose eingesetzt. Als fakultativ verwendbare weitere Kolloide kommen insbesondere schwerlösliche Polymethylmethacrylsäureester oder Macrogol, wie beispielsweise Macrogol 6000, in Betracht.

Als Hilfsstoffe können schwerlösliche Polymersubstanzen, wie Ethylcellulose, Celluloseacetat oder Polyvinylacetat mit kationischen Ammoniumgruppen, und magensaftresistente Substanzen, wie anionische Polymethacrylsäureester, Celluloseacetphthalat oder Hydroxypropylmethylcellulosephthalat, eingesetzt werden.

Die gegebenenfalls zusätzlich einzusetzenden Farb- und Aromastoffe sind dem Fachmann bekannt. Die Auswahl bestimmter Stoffe hängt von dem gewünschten Ergebnis ab und liegt im Bereich des Wissens des Fachmanns.

Die Erfindung ist nach Ansprüche 1-12 auszuführen.

Beispiel

A. Es werden zunächst Extrusionspellets nach dem Verfahren der DE-A 40 21 678 mit folgender Zusammensetzung hergestellt:

|  | g | % |
|---|---|---|
| Etofibrat | 67,2 | 84 |
| Polymethacrylsäureester mit kationischen Ammoniumgruppen (Eudragit RS 30 D - berechnet als Festsubstanz) | 8,0 | 10 |
| Triacetin | 0,8 | 1 |
| Na-Carboxymethylcellulose | 4,0 | 5 |
| Gesamtfeuchtgehalt | | 18,9 |

B. Überzugssuspension

Eine Suspension wird unter kräftigem Rühren aus den folgenden Komponenten hergestellt:

| | |
|---|---|
| Polyvinylalkohol (Mowiol 4-88) | 4,6 % |
| Polyvinylpyrrolidon, quervernetzt (Crospovidone, Kollidon CL) | 7 % |
| Cellulosepulver (Vitacel M 80 K) | 6,8 % |
| Bananenaromapulver | 1,4 % |
| Macrogol 6.000 | 0,2 % |
| Wasser | 80 % |

C. Herstellung der überzogenen Extrusionspellets

850 g der nach A. hergestellten Pellets werden in einem Dragierkessel in mehreren Schritten durch Aufsprühen mit einer Suspension gemäß B., deren Gehalt an Trockensubstanz 150 g beträgt, überzogen. Auf eine sorgfältige Trocknung zwischen den einzelnen Aufträgen ist zu achten. Die Temperatur der für die Trocknung benutzten Zuluft beträgt maximal 40°C.

D. Tablettierung

Aus den gemäß C. hergestellten, gut getrockneten überzogenen Extrusionspellets werden auf einer Exzenter- oder Rundläuferpresse Komprimate mit einem Wirkstoffgehalt von 500 mg, entsprechend 700-750 mg überzogener Extrusionspellets, unter Anwendung einer Preßkraft von 3-3,5 kN verpreßt.

Die resultierenden Komprimate haben eine Bruchfestigkeit in der ERWEKA-Apparatur von 4,5-6 kg, und die Friabilität in der ERWEKA-Apparatur - nach 5 min bei 25 UpM gemessen - beträgt 0-3 %. Der Zerfall der erfindungsgemäß hergestellten Komprimate liegt unter 30 s bei Verwendung von Wasser (20°C) als Inkubationsmedium. Die Bioverfügbarkeit des Wirkstoffs Etofibrat ist derjenigen bei Verwendung von in Hartkapseln gefüllten Etofibrat-Extrusionspellets vergleichbar.

## Patentansprüche

1. Verfahren zur Herstellung mechanisch stabiler, gut zerfallender Komprimate aus gegebenenfalls ausgerundeten, kleindimensionierten Extrusionspellets mit hohem Etofibratgehalt, dadurch gekennzeichnet, daß auf die Extrusionspellets eine wäßrige oder wäßrig-organische Suspension bestehend aus
   a) wasserlöslichem Polyvinylalkohol mit einem Estergehalt von 19,4 bis 6,7 %,
   b) einem Sprengmittel,
   c) Cellulose,
   d) gegebenenfalls weiteren Kolloiden, Hilfsstoffen sowie Farbstoffen und/oder Aromastoffen
   aufgetragen wird, die überzogenen Extrusionspellets getrocknet und dann zu Komprimaten verpreßt werden.

2. Verfahren nach Anspruch 1, dadurch gekennzeichnet, daß als Sprengmittel quervernetztes Polyvinylpyrrolidon verwendet wird.

3. Verfahren nach einem der Ansprüche 1 oder 2, dadurch gekennzeichnet, daß die Cellulose in Form von Cellulosepulver und/oder mikrokristalliner Cellulose verwendet wird.

4. Verfahren nach einem der Ansprüche 1 bis 3, dadurch gekennzeichnet, daß die Suspension 0,1 bis 20 Gew.-% Polyvinylalkohol, 0,1-20 Gew.-% Sprengmittel, 0,1-20 Gew.-% Cellulose, 0-20 Gew.-% Aroma- und/oder Farbstoffe sowie 0-20 Gew.-% andere Kolloide und/oder andere Hilfsstoffe enthält und die Gesamtmenge der Inhaltsstoffe der Suspension 1-40, vorzugsweise 15-25 Gew.-%, bezogen auf die gesamte Suspension, beträgt.

5. Verfahren nach Anspruch 4, dadurch gekennzeichnet, daß Polyvinylalkohol, Sprengmittel und Cellulose jeweils in Mengen von 4-7 Gew.-% in der Suspension enthalten sind.

6. Verfahren nach einem der Ansprüche 1 bis 5, dadurch gekennzeichnet, daß als weitere Kolloide schwerlösliche Polymethylmethacrylsäureester oder Macrogol, insbesondere Macrogol 6000, verwendet werden.

7. Verfahren nach einem der Ansprüche 1 bis 6, dadurch gekennzeichnet, daß als Hilfsstoffe magensaftresistente, filmbildende Substanzen, wie anionische Polymethacrylsäureester, Celluloseacetatphthalat oder Hydroxypropylmethylcellulosephthalat verwendet werden.

8. Verfahren nach einem der Ansprüche 1 bis 7, dadurch gekennzeichnet, daß als Hilfsstoffe schwerlösliche Polymersubstanzen, wie Ethylcellulose, Celluloseacetat, Polyvinylacetat oder Polymethacrylsäureester mit kationischen Ammoniumgruppen verwendet werden.

9. Verfahren nach einem der Ansprüche 1 bis 9, dadurch gekennzeichnet, daß das Trocknen der überzogenen Extrusionspellets bei Temperaturen bis maximal 40°C erfolgt.

10. Verfahren nach einem der Ansprüche 1 bis 9, dadurch gekennzeichnet, daß der aufgebrachte Überzug der Extrusionspellets 1 bis 60 Gew.-%, bezogen auf das überzogene Pellet, beträgt.

11. Verfahren nach einem der Ansprüche 1 bis 10, dadurch gekennzeichnet, daß die getrockneten Extrusionspellets unter Anwendung eines Druckes von 2 bis 10, vorzugsweise 2,5 bis 5 und insbesondere bevorzugt 3 bis 3,5 kN, vorzugsweise auf einer Exzenter- oder Rundläuferpresse zu Komprimaten verpreßt werden.

12. Verfahren nach einem der Ansprüche 1 bis 11, dadurch gekennzeichnet, daß die Komprimate mit einem Überzug aus einer filmbildenden Substanz, die gegebenenfalls Pigmente, Zucker oder andere Süßstoffe, Farbstoff, Aromastoffe, Gleitmittel und/oder Weichmacher enthält, versehen werden.

## Claims

1. Process for the production of mechanically stable tablets with good disintegration properties prepared from optionally rounded extruded pellets of small dimension with an elevated etofibrate content, characterised in that an aqueous or aqueous-organic suspension consisting of
   a) water-soluble polyvinyl alcohol with an ester content of 19.4 to 6.7%,
   b) a suspending agent,
   c) cellulose,
   d) optionally further colloids, auxiliary substances, together with dyes and/or flavourings,
   is applied onto the extruded pellets and the coated extruded pellets are dried and then compressed into tablets.

2. Process according to claim 1, characterised in that polyvinylpyrrolidone is used as the suspending agent.

3. Process according to one of claims 1 or 2, characterised in that the cellulose is used in the form of cellulose powder and/or microcrystalline cellulose.

4. Process according to one of claims 1 to 3, characterised in that the suspension contains 0.1 to 20 wt.% of polyvinyl alcohol, 0.1-20 wt.% of suspending agent, 0.1-20 wt.% of cellulose, 0-20 wt.% of flavourings and/or dyes, together with 0-20 wt.% of other colloids and/or other auxiliary substances and the total quantity of substances contained in the suspension constitutes to 1-40, preferably 15-25 wt.%, related to the total suspension.

5. Process according to claim 4, characterised in that the suspension contains quantities of 4-7 wt.% each of polyvinyl alcohol, suspending agent and cellulose.

6. Process according to one of claims 1 to 5, characterised in that sparingly soluble polymethyl methacrylates or macrogol, in particular macrogol 6000, are used as further colloids.

7. Process according to one of claims 1 to 6, characterised in that film-forming substances resistant to gastric juices, such as anionic polymethacrylates, cellulose acetate phthalate or hydroxypropyl methylcellulose phthalate are used as auxiliary substances.

8. Process according to one of claims 1 to 7, characterised in that sparingly soluble polymeric substances, such ethyl cellulose, cellulose acetate, polyvinyl acetate or polymethacrylates with cationic ammonium groups are used as auxiliary substances.

9. Process according to one of claims 1 to 8, characterised in that the coated extruded pellets are dried at a maximum temperature of up to 40°C.

10. Process according to one of claims 1 to 9, characterised in that the coating applied to the extruded pellets constitutes 1 to 60 wt.% related to the coated pellet.

11. Process according to one of claims 1 to 10, characterised in that the dried extruded pellets are compressed into tablets using a pressure of 2 to 10, preferably 2.5 to 5 and particularly preferably 3 to 3.5 kN, preferably on an eccentric or rotary press.

12. Process according to one of claims 1 to 11, characterised in that the tablets are provided with a coating of a film-forming substance, which optionally contains pigments, sugar or other sweeteners, dyes, flavourings, lubricants and/or plasticisers.


## Revendications

1. Procédé de production de corps comprimés mécaniquement stables, se délitant bien, à partir de granules d'extrusion de petites dimensions, arrondis le cas échéant, ayant une teneur élevée en étofibrate, caractérisé en ce qu'on applique sur les granules d'extrusion une suspension aqueuse ou organo-aqueuse composée de:
   a) poly(alcool vinylique) hydrosoluble ayant une teneur en esters de 19,4 à 6,7 %,

b) désintégrant,

c) cellulose,

d) le cas échéant d'autres colloïdes, d'adjuvants ainsi que de colorants et/ou d'arômes,

que l'on sèche les granules d'extrusion enrobés et qu'on les transforme par compression en corps comprimés.

2. Procédé selon la revendication 1, caractérisé en ce qu'on utilise comme désintégrant de la polyvinylpyrrolidone réticulée.

3. Procédé selon une des revendications 1 et 2, caractérisé en ce que la cellulose est utilisée sous forme de cellulose en poudre et/ou de cellulose microcristalline.

4. Procédé selon une des revendications 1 à 3, caractérisé en ce que la suspension contient 0,1 à 20% en poids de poly(alcool vinylique), 0,1 à 20 % en poids de désintégrant, 0,1 à 20 % en poids de cellulose, 0 à 20 % en poids d'arômes et/ou de colorants ainsi que 0 à 20 % d'autres colloïdes et/ou d'autres adjuvants et que la quantité totale des composants de la suspension représente 1 à 40 %, de préférence 15 à 25 % en poids, par rapport à la suspension totale.

5. Procédé selon la revendication 4, caractérisé en ce que la suspension contient le poly(alcool vinylique), le désintégrant et la cellulose en des quantités respectives de 4 à 7 % en poids.

6. Procédé selon une des revendications 1 à 5, caractérisé en ce qu'on utilise comme autres colloïdes des poly(méthacrylates de méthyle) peu solubles ou du Macrogol, notamment du Macrogol 6 000.

7. Procédé selon une des revendications 1 à 6, caractérisé en ce qu'on utilise comme adjuvants des substances filmogènes gastro-résistantes telles que des polyméthacrylates anioniques, de l'acétophtalate de cellulose ou du phtalate d'hydroxypropylméthylcellulose.

8. Procédé selon une des revendications 1 à 7, caractérisé en ce qu'on utilise comme adjuvants des polymères peu solubles tels que de l'éthylcellulose, de l'acétate de cellulose, du poly(acétate de vinyle) ou du polyméthacrylate porteur de groupements ammonium cationiques.

9. Procédé selon une des revendications 1 à 8, caractérisé en ce que le séchage des granules d'extrusion enrobés s'effectue à des température maximales de 40°C.

10. Procédé selon une des revendications 1 à 9, caractérisé en ce que l'enrobage appliqué sur les granules d'extrusion représente 1 à 60 % en poids, par rapport au granule enrobé.

11. Procédé selon une des revendications 1 à 10, caractérisé en ce que les granules d'extrusion séchés sont transformés en corps comprimés par application d' une pression de 2 à 10, de préférence de 2,5 à 5 et mieux encore de 3 à 3,5 kN, de préférence sur une presse à excentrique ou une presse à table tournante.

12. Procédé selon une des revendications 1 à 11, caractérisé en ce que les corps comprimés sont dotés d'un enrobage en une substance filmogène qui contient le cas échéant des pigments, des sucres ou d'autres édulcorants, des colorants, des arômes, des lubrifiants et/ou des émollients.